# FASCICULE DE BREVET EUROPEEN

(11) **EP 2 367 534 B1**
(45) Date de publication et mention de la délivrance du brevet: **01.11.2017**
(21) Numéro de dépôt: 09784344.5
(22) Date de dépôt: 19.11.2009
(51) Int. Cl.: A61K 9/16, A61K 9/20, A61K 31/167, A61K 31/192, A61K 31/343, A61K 31/7056

(54) **COMPOSITIONS PHARMACEUTIQUES UTILISANT L'INULINE COMME EXCIPIENT DE GRANULATION**
PHARMAZEUTISCHE ZUSAMMENSETZUNGEN MIT INULIN ALS GRANULATIONSTRÄGER
PHARMACEUTICAL COMPOSITIONS CONTAINING INULIN AS GRANULATION CARRIER

(30) Priorité: 19.11.2008 FR 0857850
(43) Date de publication de la demande: 28.09.2011
(73) Titulaire: Fauran, François, 1296 Coppet (CH)
(72) Inventeur: Fauran, François, 1296 Coppet (CH)
(74) Mandataire: Lavé, Michèle
(86) Numéro de dépôt international: PCT/FR2009/001328
(87) Numéro de publication internationale: WO 2010/058104

(56) Documents cités:
- EP-A- 1 825 847
- WO-A-89/00045
- WO-A-2008/007150
- WO-A-2008/061874
- WO-A1-00/76548
- US-A1- 2003 059 471
- US-B1- 6 190 687
- ANONYMOUS: "Tablet production by direct dry compression - using fructans as filler and binder" WPI WORLD PATENT INFORMATION DERWENT, DERWENT, GB, vol. 1, no. 98, 1 janvier 1900 (1900-01-01), XP002065043

## Description

La présente invention a pour objet des compositions pharmaceutiques comprenant des granulés obtenus par granulation par voie humide d'un mélange d'une substance active présentant une faible comprimabilité et d'inuline en tant que seul excipient de granulation.

De nombreuses substances à l'état solide, notamment des substances pharmaceutiquement actives, sont difficiles à mettre sous la forme de formulations solides, en particulier comprimés ou gélules. Ainsi, la compression directe de certaines substances pharmaceutiquement actives s'avère difficile voir impossible même avec des excipients spécifiques. Les problèmes de comprimabilité de ces substances actives sont principalement liés à leur faible densité, d'où une aptitude réduite à la cohésion entre les particules, et à leur faible aptitude à l'écoulement.

Par ailleurs, pour certaines substances actives dont le dosage unitaire est élevé (par exemple supérieur à 300 mg), il existe une limite quantitative à l'utilisation des excipients pour améliorer les performances rhéologiques des formulations. Cette limite est définie par une forme pharmaceutique (comprimé ou gélule) de taille convenable afin de garantir une bonne compliance au traitement.

Les compositions selon l'invention s'appliquent à des substances actives présentant une faible comprimabilité.

Afin de pouvoir être mises sous forme de comprimés ou de poudre pour gélules, ces substances actives doivent subir un traitement préalable, par exemple une granulation par voie sèche ou humide un séchage par atomisation, et être associées à des excipients, tels que liants, diluants, désintégrants, lubrifiants.

Certains documents de l'art antérieur décrivent des procédés pour faciliter la mise sous la forme de formulations galéniques solides de substances actives présentant une faible comprimabilité.

Le brevet EP0991415 décrit une forme galénique solide administrable par voie orale, obtenue par compactage d'un mélange de ribavirine et d'un désintégrant. Le compactage est réalisé avec un compacteur à rouleaux.

Le brevet US6720000 décrit un procédé pour préparer des granulés de ribavirine par granulation par voie humide d'un mélange de ribavirine et d'au moins un désintégrant, liant et charge puis extrusion.

La demande EP1351667 décrit une forme galénique solide obtenue par compactage dans un compacteur à rouleaux du citalopram et éventuellement d'excipients pharmaceutiquement acceptables.

Certains documents de l'art antérieur décrivent l'utilisation de l'inuline en tant qu'excipient dans des compositions pharmaceutiques.

Le brevet EP1128815 décrit un procédé de préparation de comprimés à croquer ou à sucer contenant un composé du calcium, par granulation par voie humide du composé du calcium avec une solution aqueuse d'un liant soluble dans l'eau, qui peut être de l'inuline. L'exemple 8 décrit des granulés obtenus par granulation de carbonate de calcium et d'inuline, puis granulation du premier granulé avec une quantité supplémentaire d'inuline et d'autres excipients.

Les documents US6190687 et WO9906028 décrivent un procédé pour déposer un extrait d'un principe actif sur les parois internes et externes d'un support microporeux se présentant sous forme de microgranulés constitués d'une fibre alimentaire soluble, à base d'un polymère, tel que l'inuline.

Les documents WO8900045 et EP1825847 décrivent des compositions pharmaceutiques obtenues par granulation par voie humide d'un mélange contenant au moins une substance active et un polymère utilisé en tant que charge. Ce polymère peut être de l'inuline, mais il n'y a aucun exemple de compositions contenant de l'inuline.

La demande WO2008007150 décrit des compositions pharmaceutiques contenant du paracétamol ou un agent anti-inflammatoire non stéroïdien obtenues par séchage par atomisation d'une solution ou dispersion de la substance active, d'un excipient et d'un solvant. L'inuline est mentionnée dans la liste des excipients, mais il n'y a aucun exemple de compositions contenant de l'inuline.

La présente demande se propose de résoudre le problème de la mise en forme de substances actives présentant une faible comprimabilité par fabrication de granulés obtenus par granulation par voie humide d'un mélange de la substance active et d'inuline en tant que seul excipient de granulation.

La granulation par voie humide est utilisée de manière classique, notamment pour les substances pharmaceutiquement actives difficiles à mettre sous la forme de formulations galéniques solides. On peut ainsi espérer préparer des granulés dans lesquels la substance active est densifiée.

Le procédé de granulation par voie humide consiste à mélanger la substance active avec au moins 2 excipients, le plus souvent un liant et un diluant, et à granuler le mélange avec un solvant de granulation.

Dans les compositions de l'invention, on utilise un seul excipient de granulation, l'inuline, qui remplit à la fois les fonctions de liant et de diluant.

L'inuline est un polyfructane d'origine naturelle dont l'innocuité et la qualité d'excipient sont parfaitement établies. C'est une fibre végétale qui n'est pas absorbée car elle n'est pas hydrolysée par les enzymes au cours du transit digestif. Outre son excellente tolérance biologique, elle est aussi un pré biotique reconnu.

L'inuline est présente dans de nombreux végétaux en particulier dans leurs tubercules dont elle constitue des substances de réserve pour leur croissance, par exemple les végétaux de la famille des composées comme Dahlia variabilis et Helianthus tuberosus (topinambour). La source industriellement la plus utilisée est polymérisation moyen varie en fonction de l'espèce végétale, pour la chicorée le degré de polymérisation moyen n est égal à 10.

Les compositions de la présente invention contiennent des substances actives présentant une faible comprimabilité. A titre d'exemples non limitatifs, on peut citer la ribavirine, l'ibuprofène, le paracétamol et le citalopram.

Les compositions sont obtenues par granulation par voie humide d'un mélange d'inuline, de substance active et d'un solvant de granulation pharmaceutiquement acceptable. Le rapport en masse entre l'inuline et la substance active est compris entre 0,1 et 0,4, de préférence entre 0,2 et 0,3, tout particulièrement il est égal à 0,26. Comme solvant de granulation, on peut utiliser l'eau ou les mélanges eau/alcool. On utilise de préférence de l'eau en une quantité égale à 5 à 20% de la masse des poudres. Les granulés obtenus sont calibrés et séchés.

Après granulation, on peut ajouter en phase externe des excipients pharmaceutiquement acceptables, tels que des désintégrants et/ou des lubrifiants. De préférence, on utilise un désintégrant, tel que la croscarmellose, et un lubrifiant, tel que le stéarate de magnésium. La quantité de désintégrant est comprise entre 1 et 5%, de préférence entre 2 et 3%, en particulier elle est égale à 2% en masse sur la base de la masse des granulés. La quantité de lubrifiant est comprise entre 0,5 et 3%, de préférence entre 1 et 2%, en particulier elle est égale à 1% en masse sur la base de la masse des granulés.

Les granulés peuvent être répartis dans des gélules ou dans des sachets ou mis sous forme de comprimés.

Les granulés présentent une teneur élevée en substance active, par exemple de 60 à 85%, ce qui permet d'obtenir des formes galéniques améliorant la compliance du patient à son traitement.

Par exemple, dans le cas de la ribavirine, on peut utiliser des gélules d'une taille inférieure (taille 2 pour 200 mg de ribavirine) à celles actuellement sur le marché (Gélules Rébétol® 200 mg de taille 1). On peut envisager un dosage unitaire de 400 mg dans des gélules de taille 0 et un dosage unitaire de 600 mg dans des gélules de taille 00.

La compression des granulés permet également de préparer des comprimés dosés par exemple à 200, 300, 400 ou 600 mg de substance active présentant des caractéristiques satisfaisantes. Par exemple, on a obtenu, à partir d'un lot de 15 kg de granulés, des comprimés contenant 400 mg de ribavirine d'une masse moyenne de 520 mg, d'une dureté de 140 N et des comprimés contenant 600 mg de ribavirine d'une masse moyenne de 780 mg, d'une dureté de 130 N.

### Exemple 1 : Etude de la quantité d'inuline dans les granulés

Dans cet exemple, la substance active utilisée est la ribavirine présentant une densité de 0,23 g/ml.

Afin de déterminer la quantité optimale d'inuline dans les compositions de l'invention, nous avons étudié la densité et l'aptitude à la compression du granulé en fonction du rapport inuline/ribavirine.

Les études ont été faites sur des lots de taille laboratoire (175 à 500 g).

Les volumes apparents avant tassement (V0) et après x tassements (Vx) ont été mesurés selon la méthode de la Pharmacopée Européenne (EP 2.9.15) et les densités correspondantes calculées à partir du poids de l'échantillon.

La densité du granulé, D0 avant tassement, Dx après x tassements ou Dt densité tapée, est exprimée en g/ml.

On mesure l'aptitude à la compression du granulé à l'aide de l'indice de Carr (indice de compressibilité), qui est égal à 100 × (Dt - D0)/Dt. Plus l'indice de Carr est faible, plus la compressibilité est bonne. On considère que l'indice de Carr est satisfaisant pour une valeur de 15 à 20.

On peut également utiliser l'aptitude au tassement selon la Pharmacopée Européenne (EP 2.9.15), V₁₀ - V₅₀₀, qui est la différence entre le volume après 10 chutes et le volume après 500 chutes de l'éprouvette. Cette différence est exprimée en mL ; on considère que l'aptitude au tassement est satisfaisante lorsque V₁₀ - V₅₀₀ est inférieur ou égal à 20 mL.

| | | | | | |
|---|---|---|---|---|---|
| Inuline/ribavirine | 0 | 0,055 | 0,115 | 0,26 | 0,41 |
| Densité D0 (g/ml) | 0,23 | 0,38 | 0,46 | 0,54 | 0,6 |
| Indice de Carr | 41 | 32,1 | 18,8 | 18,4 | 16,7 |

Les études ont montré que la densification de la ribavirine est une fonction logarithmique du rapport inuline/ribavirine. Pour un rapport inuline/ribavirine égal à 0,26 on obtient une densité très satisfaisante de 0,54 g/ml.

L'indice de Carr est égal à 32,1 pour un rapport inuline/ribavirine de 0,055, il décroît ensuite très rapidement pour atteindre une valeur acceptable dès un rapport inuline/ribavirine de 0,115.

Compte tenu des résultats précédents, il apparaît qu'un rapport inuline/ribavirine égal à 0,26 donne de bons résultats en ce qui concerne la densité et l'indice de Carr, tout en permettant de minimiser la quantité d'inuline et d'optimiser ainsi la taille de la forme galénique finale.

Etant donné que les granulés préférés selon l'invention contiennent également un désintégrant, tel que la croscarmellose, et un lubrifiant, tel que le stéarate de magnésium, la composition optimale des granulés est la suivante :
- substance active 77%
- inuline 20%
- croscarmellose 2%
- stéarate de magnésium 1%.

### Exemple 2 : Granulés de ribavirine

En utilisant la composition optimale déterminée dans l'exemple 1, on a préparé 8 kg de granulés de ribavirine de la manière suivante :
Etape 1 : on a mélangé la ribavirine (6154 g) présentant une densité de 0,23 g/ml et l'inuline (1606 g) dans un mélangeur de type Diosna pendant 3 minutes.
Etape 2 : dans le même mélangeur, on a opéré une granulation par addition progressive d'eau purifiée (956 g) représentant une quantité d'environ 12% en masse par rapport au mélange ribavirine/inuline.
Etape 3 : on a séché les granulés sur lit d'air fluidisé (Glatt) à une température de 40°C pendant 10 minutes puis à 45°C jusqu'à obtention de granulés présentant une humidité résiduelle de 1,8%.
Etape 4 : on a calibré les granulés sur un appareil Quadro Comi pour donner des granulés, dont plus de 50% ont un diamètre inférieur à 250 µm et 70% ont un diamètre inférieur à 100 µm.
Etape 5 : on a mélangé les granulés et la croscarmellose sodique (160 g) dans un mélangeur de type Bohle ou Englesman, pendant au moins 10 minutes.
Etape 6 : dans ce même mélangeur, on a ajouté le stéarate de magnésium (80 g) et on a mélangé pendant au moins 5 minutes.

Le tableau ci-dessous indique les résultats obtenus.

| | Matière première | Granulé |
|---|---|---|
| Densité D0 (g/ml) | 0,23 | 0,75 |
| Indice de Carr | 41 | 17,2 |
| Vitesse écoulement (secondes) | Pas d'écoulement | 5 |

On a observé une bonne densification de la ribavirine (+ 226%) et une optimisation des paramètres d'écoulement et d'aptitude à la compression.

### Exemple 3 : Granulés de ribavirine utilisant différentes inulines

On a préparé des granulés selon le mode opératoire de l'exemple 2 en utilisant différentes qualités d'inuline. Il s'agit d'inulines extraites de chicorée obtenues auprès de Beneo-Orafti et présentant des degrés de polymérisation de 8 à 23 avec des teneurs en inuline de 92 à 100%.

Le tableau ci-dessous indique les caractéristiques des inulines utilisées.

| Inuline Beneo-Orafti® | GR | LGI | HP |
|---|---|---|---|
| Degré de polymérisation n | ≥ 10 | ≥ 8 | > 23 |
| Inuline (%) | 92 | >96 | 100 |

On a préparé des granulés contenant environ 20% de chacune des inulines ci-dessus avec une ribavirine présentant une densité de 0,39 ou 0,40 g/ml.

Le tableau ci-dessous indique les résultats obtenus.

| Qualité inuline | GR | LGI | HP |
|---|---|---|---|
| Densité ribavirine (g/ml) | 0,39 | 0,40 | 0,40 |
| Densité D0 du granulé (g/ml) | 0,58 | 0,54 | 0,51 |
| Vitesse écoulement (secondes) | 14 | 9 | 7 |
| Dureté après compression | 84-90 N | 90-120 N | 107 N |

La dureté a été mesurée après compression sous 0,5 tonne de 350 mg de granulés.

Quelle que soit la qualité de l'inuline retenue, on obtient un granulé densifié qui s'écoule et se comprime correctement.

### Exemple 4 : Granulés d'ibuprofène

En suivant le mode opératoire de l'exemple 2, on a préparé des granulés contenant 77% d'ibuprofène présentant une densité de 0,31 g/ml, 20% d'inuline, 2% de croscarmellose et 1% de stéarate de magnésium.

Le tableau ci-dessous indique les résultats obtenus.

| | Matière première | Granulé |
|---|---|---|
| Densité D0 (g/ml) | 0,31 | 0,52 |
| Indice de Carr | 41,5 | 10,3 |
| Vitesse écoulement (secondes) | Pas d'écoulement | 9 |

On a observé une bonne densification de l'ibuprofène (+ 68%) et une optimisation des paramètres d'écoulement et d'aptitude à la compression.

### Exemple 5 : Granulés de paracétamol

En suivant le mode opératoire de l'exemple 2, on a préparé des granulés contenant 77% de paracétamol présentant une densité de 0,34 g/ml, 20% d'inuline, 2% de croscarmellose et 1% de stéarate de magnésium.

Le tableau ci-dessous indique les résultats obtenus.

| | Matière première | Granulé |
|---|---|---|
| Densité D0 (g/ml) | 0,34 | 0,57 |
| Indice de Carr | 24,4 | 14,2 |
| Vitesse écoulement (secondes) | Pas d'écoulement | 7 |

On a observé une bonne densification du paracétamol (+ 68%) et une optimisation des paramètres d'écoulement et d'aptitude à la compression.

### Exemple 6 : Granulés de citalopram

En suivant le mode opératoire de l'exemple 2, on a préparé des granulés contenant 77% de bromhydrate de citalopram présentant une densité de 0,24 g/ml, 20% d'inuline, 2% de croscarmellose et 1% de stéarate de magnésium.

Le tableau ci-dessous indique les résultats obtenus.

| | Matière première | Granulé |
|---|---|---|
| Densité D0 (g/ml) | 0,24 | 0,57 |
| Indice de Carr | 42,9 | 13,6 |
| Vitesse écoulement (secondes) | Pas d'écoulement | 4 |

On a observé une bonne densification du citalopram (+ 137%) et une optimisation des paramètres d'écoulement et d'aptitude à la compression.

### Exemple 7 : Gélules

On a réparti les granulés obtenus selon les exemples 2, 4, 5 ou 6 dans des gélules à l'aide d'une géluleuse de type Bosch. Dans le cas de la ribavirine, on utilise des gélules de taille 2 pour un dosage de 200 mg, de taille 0 pour un dosage de 400 mg et de taille 00 pour un dosage de 600 mg.

### Exemple 8 : Comprimés

On a préparé des comprimés à partir des granulés obtenus selon les exemples 2, 4, 5 ou 6. On a utilisé une comprimeuse de type Manesty. Pour obtenir un dosage de 200 mg en substance active, on utilise 260 mg ± 5% de granulés, pour un dosage de 400 mg 520 mg ± 5% et pour un dosage 600 mg 780 mg ± 5%.

Par exemple, on a obtenu des comprimés dosés à 400 mg de ribavirine d'un poids moyen de 520 mg et de dureté égale à 140 N pour une pression de 3,15 tonnes. Les paramètres de désagrégation (7 minutes) et de dissolution dans l'eau (> 90% en 15 minutes) sont très satisfaisants.

On procède éventuellement à un pelliculage des comprimés obtenus.

### Exemple 9 : Lot industriel de comprimés de ribavirine

On a préparé un lot de taille industrielle de 41 kg de granulés de ribavirine (lot B3) selon le mode opératoire de l'exemple 2, en utilisant une matière première d'une densité égale à 0,4 mg/ml et une inuline HP, et après adaptation des équipements à la taille considérée. Les granulés obtenus ont ensuite été comprimés sur une rotative Betapress F16.

La densification de la poudre (densité D0) ainsi que sa comprimabilité (indice de Carr et V₁₀ - V₅₀₀) ont été mesurées aux différentes étapes de la fabrication du granulé.

Le tableau ci-dessous indique les résultats obtenus.

| Lot B3 | V₁₀ - V₅₀₀ ml | Indice de Carr | D0 mg/ml |
|---|---|---|---|
| Matière première | 86,0 | 37,5 | 0,4 |
| Mélange physique | 56,0 | 32 | 0,45 |
| Granulé | 14,0 | 20 | 0,68 |
| Granulé + phase externe | 20,0 | 19 | 0,72 |

Ces résultats montrent que
- la seule utilisation d'une matière première de densité 0,4 mg/ml, supérieure à celle utilisée dans l'exemple 2, ne permet pas d'obtenir les paramètres rhéologiques requis et ce même après mélange physique avec l'inuline,
- c'est bien la seule opération de granulation qui est à l'origine de l'amélioration de la densification et de la comprimabilité du granulé. En effet l'addition de la phase externe n'améliore pas significativement les paramètres obtenus.

On procède ensuite à un pelliculage des comprimés obtenus en utilisant une turbine Pellegrini par pulvérisation d'une suspension d'Opadry®, la couleur étant adaptée pour chaque dosage unitaire, jusqu'à obtention d'une augmentation de la masse de 3%.

On a également testé les propriétés de ces comprimés pelliculés.

Ils satisfont au test de l'uniformité de teneur des préparations unidoses de la Pharmacopée Européenne (EP 2.9.40) et de l'uniformité de masse (EP 2.9.5).

On a étudié leur dissolution dans les conditions définies par la Pharmacopée Européenne avec un appareil de type 2, appareil à palettes, en utilisant l'eau comme milieu et dans les conditions les plus discriminantes : volume 500 ml et agitation 50 tours par minute.

Le tableau ci-dessous indique les résultats obtenus.

| Temps minutes | % dissous | écart-type |
|---|---|---|
| 5 | 35 | 6 |
| 10 | 73 | 8,6 |
| 15 | 92 | 6 |
| 30 | 100 | 1,8 |
| 45 | 100 | 1,5 |

Les résultats, moyenne pour 12 comprimés, montrent la rapidité de la dissolution de la ribavirine : plus de 90 % en 15 minutes. Dès lors les comprimés répondent à la définition d'une forme orale à libération immédiate (75% dissous en 45 minutes).

## Revendications

1. Compositions pharmaceutiques **caractérisées en ce qu'**elles comprennent des granulés obtenus par granulation par voie humide d'un mélange d'une substance pharmaceutiquement active présentant une faible comprimabilité, d'inuline en tant que seul excipient de granulation et d'un solvant de granulation pharmaceutiquement acceptable, le rapport en masse entre l'inuline et la substance active étant compris entre 0,1 et 0,4, permettant d'obtenir une densification importante de la substance active, et lesdites compositions comprenant de 60 à 85% de substance active.

2. Compositions pharmaceutiques selon la revendication 1, **caractérisées en ce que** le rapport en masse entre l'inuline et la substance active est égal à 0,26.

3. Compositions pharmaceutiques selon l'une quelconque des revendications précédentes **caractérisées en ce qu'**elles comprennent en outre des excipients pharmaceutiquement acceptables.

4. Compositions pharmaceutiques selon l'une quelconque des revendications précédentes **caractérisées en ce que** les excipients pharmaceutiquement acceptables sont choisis parmi les lubrifiants et les désintégrants.

5. Compositions pharmaceutiques selon l'une quelconque des revendications précédentes **caractérisées en ce qu'**elles comprennent du stéarate de magnésium en tant que lubrifiant.

6. Compositions pharmaceutiques selon l'une quelconque des revendications précédentes **caractérisées en ce qu'**elles comprennent de la croscarmellose en tant que désintégrant.

7. Compositions pharmaceutiques selon l'une quelconque des revendications précédentes, **caractérisées en ce que** la substance active est choisie parmi la ribavirine, l'ibuprofène, le citalopram ou le paracétamol.

8. Compositions pharmaceutiques selon l'une quelconque des revendications précédentes, **caractérisées en ce qu'**elles se présentent sous forme de comprimés, de gélules ou de poudre.

9. Compositions pharmaceutiques selon l'une quelconque des revendications précédentes, **caractérisées en ce que** la substance active est la ribavirine.

10. Compositions pharmaceutiques selon la revendication 9, **caractérisées en ce qu'**elles contiennent de 200 mg à 600 mg de ribavirine.

## Patentansprüche

1. Pharmazeutische Zusammensetzungen, **dadurch gekennzeichnet, dass** sie Granulate enthalten, die durch Nassgranulation eines Gemischs aus einem pharmazeutischen Wirkstoff, der eine geringe Tablettierfähigkeit aufweist, Inulin als einzigem Granulationsexzipienten und einem pharmazeutisch annehmbaren Granulationslösungsmittel erhalten werden, wobei das Massenverhältnis zwischen Inulin und dem Wirkstoff zwischen 0,1 und 0,4 beträgt, wodurch eine erhebliche Verdichtung des Wirkstoffs erhalten werden kann, und die Zusammensetzungen 60 bis 85% Wirkstoff umfassen.

2. Pharmazeutische Zusammensetzungen nach Anspruch 1, **dadurch gekennzeichnet, dass** das Massenverhältnis zwischen Inulin und dem Wirkstoff gleich 0,26 ist.

3. Pharmazeutische Zusammensetzungen nach einem der vorhergehenden Ansprüche, **dadurch gekennzeichnet, dass** sie darüber hinaus pharmazeutisch annehmbare Exzipienten umfassen.

4. Pharmazeutische Zusammensetzungen nach einem der vorhergehenden Ansprüche, **dadurch gekennzeichnet, dass** die pharmazeutisch annehmbaren Exzipienten aus Gleitmitteln und Sprengmitteln ausgewählt sind.

5. Pharmazeutische Zusammensetzungen nach einem der vorhergehenden Ansprüche, **dadurch gekennzeichnet, dass** sie Magnesiumstearat als Gleitmittel umfassen.

6. Pharmazeutische Zusammensetzungen nach einem der vorhergehenden Ansprüche, **dadurch gekennzeichnet, dass** sie Croscarmellose als Sprengmittel umfassen.

7. Pharmazeutische Zusammensetzungen nach einem der vorhergehenden Ansprüche, **dadurch gekennzeichnet, dass** der Wirkstoff aus Ribavirin, Ibuprofen, Citalopram oder Paracetamol ausgewählt ist.

8. Pharmazeutische Zusammensetzungen nach einem der vorhergehenden Ansprüche, **dadurch gekennzeichnet, dass** sie in Form von Tabletten, Kapseln oder Pulver dargereicht werden.

9. Pharmazeutische Zusammensetzungen nach einem der vorhergehenden Ansprüche, **dadurch gekennzeichnet, dass** der Wirkstoff Ribavirin ist.

10. Pharmazeutische Zusammensetzungen nach Anspruch 9, **dadurch gekennzeichnet, dass** sie 200 mg bis 600 mg Ribavirin enthalten.

## Claims

1. Pharmaceutical compositions, **characterized in that** they comprise granules obtained by wet granulation of a mixture of a pharmaceutically active substance having a low degree of compressibility, of inulin as only granulation excipient and of a pharmaceutically acceptable granulation solvent, the weight ratio between the inulin and the active substance being between 0.1 and 0.4, making it possible to obtain significant densification of the active substance, and said compositions comprising from 60 to 85% of active substance.

2. Pharmaceutical compositions according to Claim 1, **characterized in that** the weight ratio between the inulin and the active substance is equal to 0.26.

3. Pharmaceutical compositions according to either one of the preceding claims, **characterized in that** they also comprise pharmaceutically acceptable excipients.

4. Pharmaceutical compositions according to any one of the preceding claims, **characterized in that** the pharmaceutically acceptable excipients are chosen from lubricants and disintegrants.

5. Pharmaceutical compositions according to any one of the preceding claims, **characterized in that** they comprise magnesium stearate as lubricant.

6. Pharmaceutical compositions according to any one of the preceding claims, **characterized in that** they comprise croscarmellose as disintegrant.

7. Pharmaceutical compositions according to any one of the preceding claims, **characterized in that** the active substance is chosen from ribavirin, ibuprofen, citalopram or paracetamol.

8. Pharmaceutical compositions according to any one of the preceding claims, **characterized in that** they are in the form of tablets, gel capsules or powder.

9. Pharmaceutical compositions according to any one of the preceding claims, **characterized in that** the active substance is ribavirin.

10. Pharmaceutical compositions according to Claim 9, **characterized in that** they contain from 200 mg to 600 mg of ribavirin.
